# FASCICULE DE BREVET EUROPEEN

(11) **EP 0 537 166 B1**
(45) Date de publication et mention de la délivrance du brevet: **07.07.1999**
(21) Numéro de dépôt: 91909065.4
(22) Date de dépôt: 17.04.1991
(51) Int. Cl.: C12N 15/12, C12N 15/62, C07K 14/715, A61K 38/17, G01N 33/68

(54) **POLYPEPTIDES HYDROSOLUBLES AYANT UNE HAUTE AFFINITE POUR LES INTERFERONS ALPHA ET BETA**
NEUE WASSERLÖSLICHE POLYPEPTIDE MIT HOHER AFFINITÄT FÜR ALPHA- UND BETA- INTERFERONE
WATER-SOLUBLE POLYPEPTIDES HAVING HIGH AFFINITY FOR INTERFERONS ALPHA AND BETA

(43) Date de publication de la demande: 21.04.1993
(73) Titulaire: MEDISUP INTERNATIONAL N.V., Curacao, Netherlands Antilles (AN)
(72) Inventeur: EID, Pierre, F-75013 Paris (FR); GRESSER, Ion, F-75017 Paris (FR); LUTFALLA, Georges, F-75007 Paris (FR); MEYER, François, F-94801 Villejuif Cédex (FR); MOGENSEN, Knud, Erik, F-75014 Paris (FR); TOVEY, Michael, F-75005 Paris (FR); UZE, Gilles, F-75014 Paris (FR)
(74) Mandataire: Rinuy, Santarelli
(86) Numéro de dépôt international: FR9100318
(87) Numéro de publication internationale: WO9218626

(56) Documents cités:
- FR-A- 2 657 881
- PROCEEDINGS OF THE NATL. ACADEMY OF SCIENCES USA, vol. 80, May 1983, Washington, DC (US); H. ARNHEITER et al., pp. 2539-2543
- IMMUNOLOGIE, 3e édition, De Boeck Université; I.M. ROITT et al., pp. 6.6-6.14
- CELL; vol. 60, 26 janvier 1990; G. UZE et al., pp. 225-234
- PROCEEDINGS OF THE NATL. ACADEMY OF SCIENCES USA, vol. 80, juin 1983, Washington, DC (US); C.R. FALTYNEK et al., pp. 3269-3272

## Description

La présente invention concerne de nouveaux polypeptides hydrosolubles, des séquences d'ADN, de nouvelles cellules, le procédé de préparation desdits polypeptides, leur application à titre de médicaments et les compositions les renfermant.

Les interférons α et β forment un groupe de protéines sécrétées douées de propriétés biologiques diverses et caractérisées par leur capacité à induire, dans des cellules de vertébrés, un état antiviral et anti-prolifératif (I. Gresser et M.G. Tovey, Biochem. Biophys. Acta 516:23/1978).

L'interféron a a des effets importants sur le système immun, cellulaire et humoral, notamment sur l'activation polyclonale des cellules B (M. Peters, J. Immunol., 137:3153/1986), l'inhibition des fonctions des cellules T (J. Knop et al, J. Immunol., 133:2412/1984) et la modulation de l'expression des antigènes d'histocompatibilité (M. Fellous et al, Eur. J. Immunol., 9:446/1979). Tous ces processus sont impliqués dans le développement de l'auto-immunité.

Alors que l'interféron est considéré comme un facteur bénéfique pour l'organisme, une production anormale d'interféron peut contribuer à la pathogénie de certaines maladies et en fait est associée à diverses maladies dites auto-immunes. Par exemple, des taux élevés d'interféron sont présents dans le sérum ou les tissus de malades atteints de diverses maladies, telles que le Lupus érythémateux, l'arthrite rhumatoïde, la maladie de Behcet, le diabète mellitus, la sclérose en plaques, l'aplasie de la moelle et une grave maladie immunodéficitaire multiple. Une corrélation directe existe entre les taux de cet interféron α et le pronostic grave de l'évolution de la maladie du SIDA (E. Buimovivi-Klein et al, AIDS Res., 2:99/ 1986).

Il a été montré que chez une souche particulière de souris (NZB) atteinte d'une maladie spontanée, maladie qui sert de modèle animal au Lupus érythémateux chez l'homme, l'administration d'interféron α ou β aggrave la progression de la maladie (H. Heremans et al, Infect. immun., 21:925/1978 ; C. Adam et al, Clin. Exp. Immunol., 40:373/1980).

Chez les souriceaux, l'administration de quantités importantes d'interféron induit un syndrome d'inhibition de croissance, nécrose du foie et mortalité (I. Gresser et al, Nature, 258:76/1975). De même, l'infection par certains virus (tels que les virus de la chorioméningite lymphocytaire Pichinde, ou rhéovirus) dans la période néonatale de la souris s'accompagne de la production de grandes quantités d'interféron endogène qui entraîne le même syndrome létal. L'administration d'un anticorps anti-interféron α ou β protège les souriceaux infectés à la naissance par les virus de ce syndrome cités ci-dessus (Y. Rivière et al, Proc. Natl. Acad. Sci. USA, 74:2135/1977 ; Y. Rivière et al, J. Exp. Med., 152:633/ 1980 ; T. Clark et al, J. Virol, 59:728/1986). Cette expérience représente un argument convaincant du rôle nocif de l'interféron dans la pathogénèse de cette maladie.

Par ailleurs, l'activation des cellules NK et la modulation de l'expression des antigènes d'histocompatibilité, sont toutes les deux régulées par l'interféron α ou β et jouent un rôle important dans le rejet des greffes de la moelle osseuse (C. Ohien et al, Science, 246:666/ 1989). En effet, il a été démontré que la production d'interféron α ou β est un des éléments essentiels dans la résistance des souris hybrides F1, à une greffe de la moelle parentale (Afifi et al, J. Immunol., 134:3739/1985). Ainsi, le traitement des souris F1 ou même des souris allogéniques par un sérum anti-interféron α ou β murin, permet la greffe et la prolifération de la moelle parentale ou allogénique (Afifi et al, 1985).

On sait aussi que les effets biologiques des interférons et de leurs sous-types sont générés par l'interaction de ceux-ci avec un récepteur spécifique de haute affinité à la surface cellulaire (M. Aguet et K.E. Mogensen, Academic Press, London, 1983).

Il n'existe actuellement aucune thérapie efficace contre les maladies auto-immunes et contre d'autres maladies telle que la sclérose en plaques, soupçonnée d'avoir une parenté avec des maladies auto-immunes. Les traitements actuels des maladies de type auto-immunes sont insatisfaisantes et ont des effets toxiques. Ceux employés dans les thérapies "anti-rejet" inhibent les manifestations de ces pathologies mais pas leurs causes et ont une toxicité très élevée. Il serait donc hautement souhaitable de disposer de médicaments ayant des effets thérapeutiques et une toxicité réduite sur les maladies auto-immunes et les rejets d'organes.

Il serait donc très souhaitable de bloquer l'action de l'interféron (α ou β) par l'injection d'un antagoniste qui ainsi pourrait être thérapeutiquement bénéfique pour les maladies de type auto-immunes et pour empêcher le rejet des greffes. Seulement, une telle approche basée sur l'injection d'immunoglobuline étrangère qui a prouvé son efficacité n'est pas pratique dans un traitement thérapeutique chez l'homme.

C'est pourquoi la présente demande concerne une nouvelle approche qui est basée sur l'utilisation d'une forme soluble du récepteur spécifique de l'interféron α comme antagoniste pour bloquer l'action de l'interféron α ou β. Ces variantes du récepteur naturel préparées par des techniques de génie génétique conservent la capacité de fixer l'interféron α endogène soit circulant, soit localement, sont dépourvues de la partie qui les fixent à la surface cellulaire ; elles peuvent circuler librement et grâce à leur spécificité ne se lient qu'à l'interféron α ou β.

Par la fixation de l'interféron α ou β, elles sont capables de bloquer -comme le ferait un anticorps-l'action de l'interféron α ou β dans l'organisme.

Il serait donc souhaitable de disposer d'un produit capable de bloquer l'activité de l'interféron α et/ou β.

C'est pourquoi la présente demande a pour objet un polypeptide hydrosoluble, caractérisé en ce qu'il a une haute affinité pour les interférons α et β.

Par "haute affinité", l'on entend une constante de dissociation inférieure à 10⁻⁹ M.

Par "hydrosoluble", l'on entend que ledit polypeptide est capable de circuler dans un organisme tel que le corps humain puis de se fixer à une cellule.

Dans la présente demande, et dans ce qui suit, par "hybride", l'on entend le produit résultant de la fusion (ou couplage) d'un polypeptide hydrosoluble selon la présente invention (partie "soluble" du récepteur naturel, récepteur complet modifié, ou partie "soluble" du récepteur naturel modifié par exemple par substitution) et d'une autre molécule, notamment de nature polypeptidique, telle qu'une immunoglobuline ou un fragment d'immunoglobuline.

Par "récepteur soluble des interférons α et β" (ou de l'interféron), l'on entend l'un des polypeptides hydrosolubles tels que définis ci-dessus.

Afin de simplifier la rédaction, dans ce qui suit, on parlera généralement de "récepteur de l'interféron" en lieu et place de "récepteur des interférons α et β".

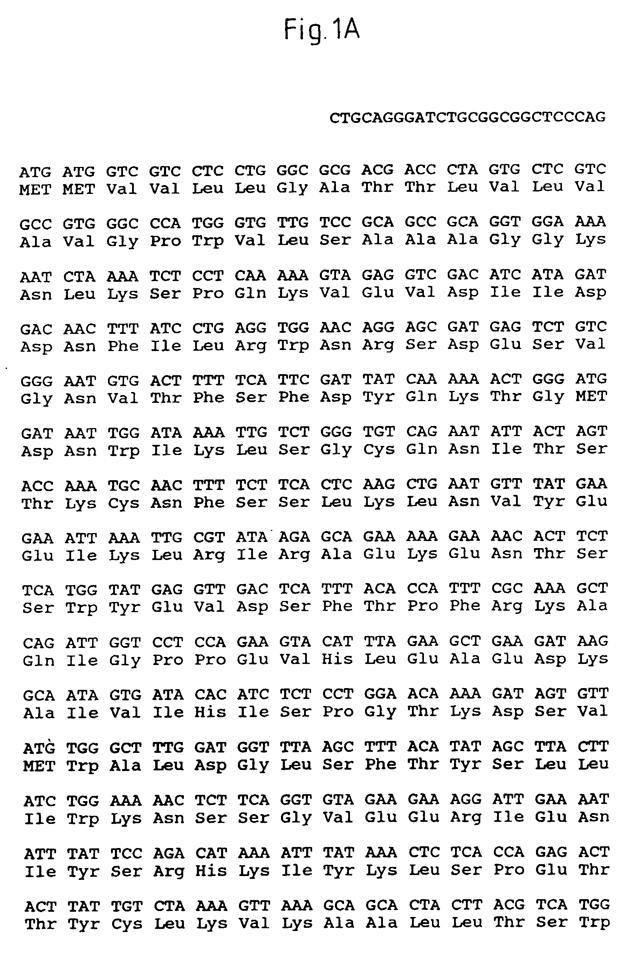

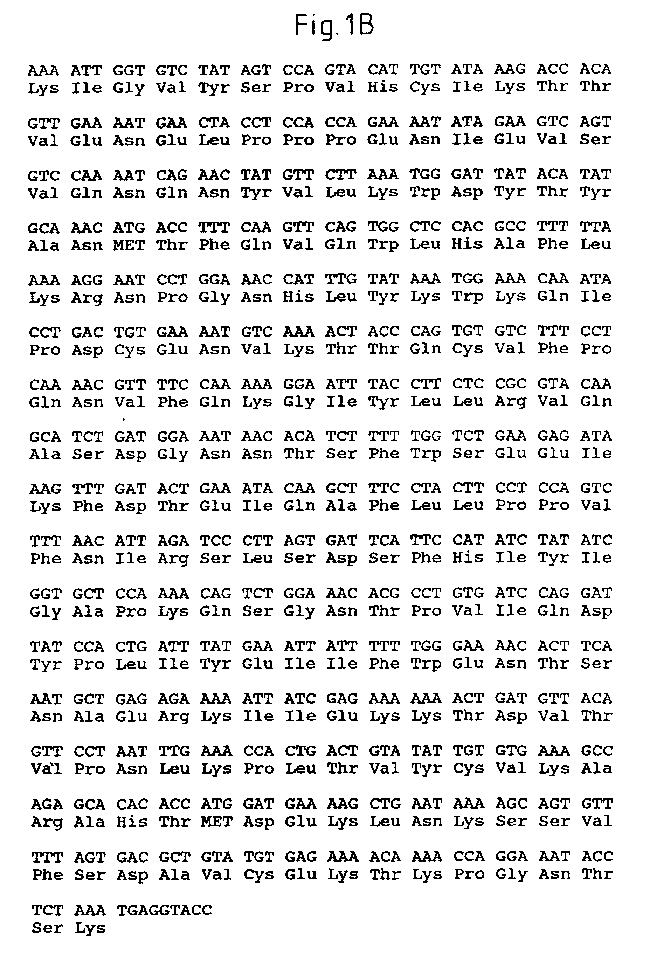

Parmi les polypeptides tels que définis ci) dessus, l'invention a notamment pour objet un polypeptide hydrosoluble, caractérisé en ce qu'il répond à la formule représentée à la figure 1.

Ce polypeptide correspond à la partie soluble, extra-cellulaire, du récepteur natif naturel de l'interféron α ou β.

Bien entendu, d'autres polypeptides que le polypeptide ci-dessus décrit conservent une haute affinité pour lesdits interférons. C'est ainsi que le polypeptide représenté à la figure 1 pourra être remplacé notamment par des variantes de substitution ou de délétion qui font également partie de l'objet de la présente demande.

En ce qui concerne les délétions, un ou plusieurs acides aminés du polypeptide répondant à la figure 1 pourront être supprimés sans modification défavorable de l'affinité vis-à-vis des interférons α et β.

Des délétions pourront concerner également le récepteur complet et natif, notamment au niveau de sa partie soluble, de manière par exemple à lui faire perdre sa capacité à se fixer à la membrane cellulaire et donc le rendre disponible dans la circulation.

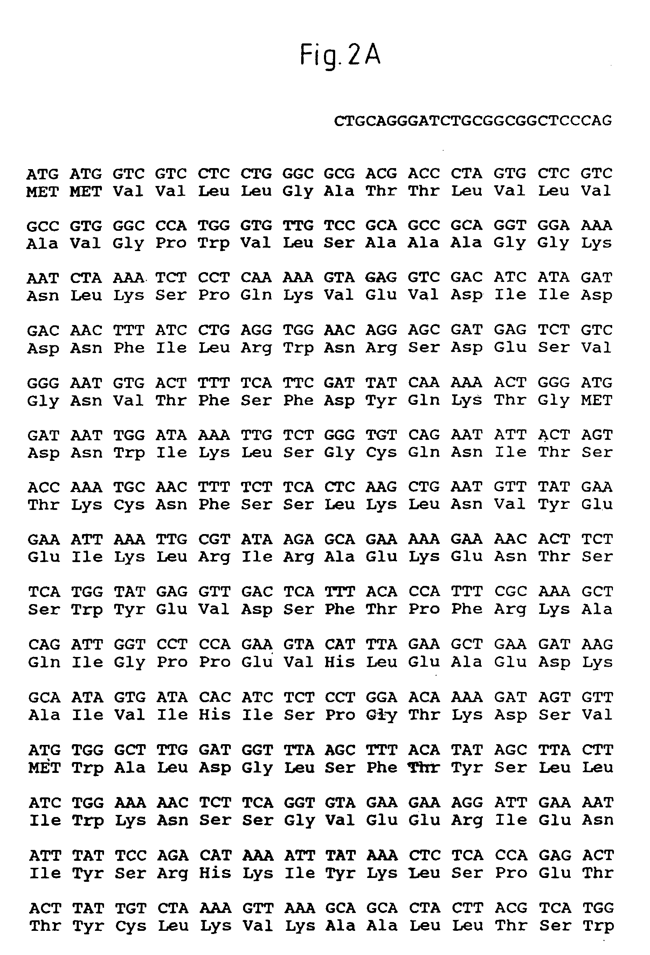

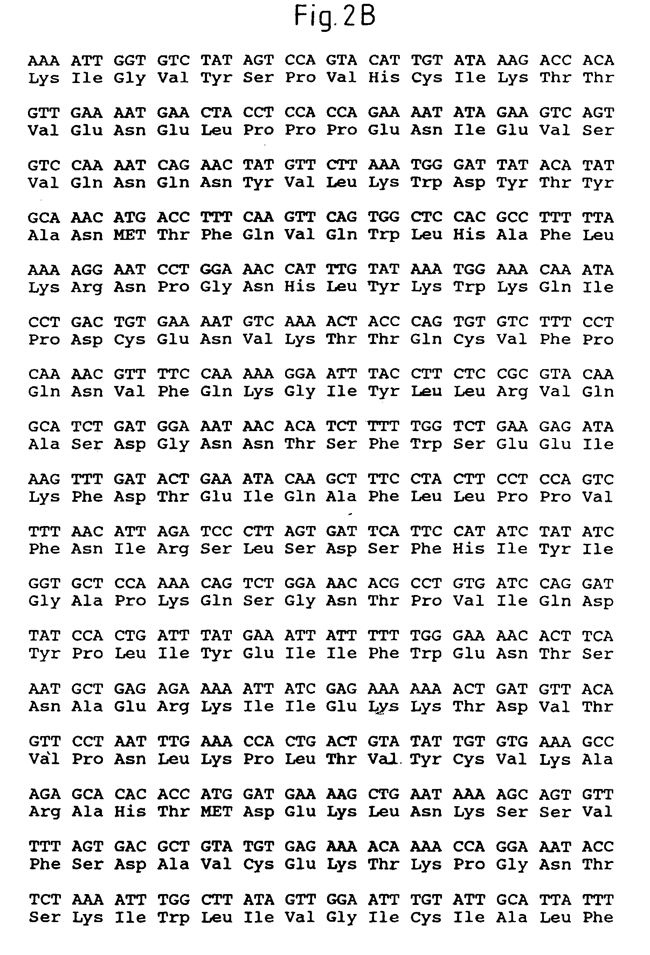

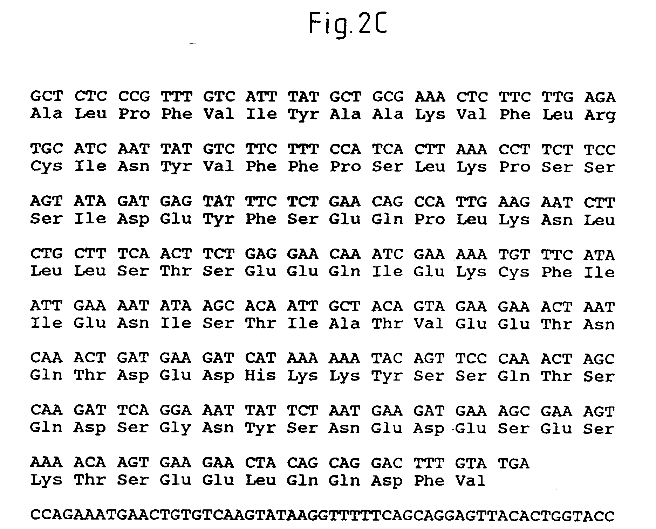

Si l'on part de la séquence du récepteur natif et complet de l'interféron représenté à la figure 2, on pourra par exemple supprimer les domaines trans-membranaire et cytoplasmique de sa séquence.

La délétion des résidus 437-457 correspondant à la région trans-membranaire et des résidus 458-557 (région cytoplasmique) sera par exemple réalisée pour obtenir des formes solubles (circulantes).

La séquence complète des acides aminés et des nucléotides codant pour le récepteur complet des interférons α et β est représentée à la figure 2.

Dans le cas où l'on effectue des délétions dans les domaines trans-membranaire et cytoplasmique (ou cellulaire et intra-cellulaire), on peut éviter des épitopes potentiellement immuns. Un avantage du récepteur natif des interférons α et β à qui on a supprimé la région trans-membranaire, est son aptitude à être sécrété dans le surnageant du milieu de culture des cellules-hôtes recombinantes.

La présente demande a donc aussi pour objet les polypeptides hydrosolubles, caractérisés en ce qu'ils dérivent par délétion des polypeptides répondant à la formule de la figure 1 ou de la figure 2.

Les variantes de substitution font également partie de l'objet de la présente demande.

Dans ce cas, un ou plusieurs acides aminés dans la séquence du récepteur de l'interféron pourront être enlevés et remplacés par d'autres, le nombre total d'acides aminés étant conservé.

Les substitutions concerneront de préférence la partie soluble du récepteur de l'interféron. Les changements substantiels dans la fonction ou d'identité immunologique de la partie soluble du récepteur seront réalisés en sélectionnant les substitutions non conservatives ainsi que des résidus d'acides aminés ou séquences qui diffèrent de ceux présents à l'origine sur le polypeptide de la manière la plus significative dans leur propriété de maintenir la structure tridimensionnelle du polypeptide dans le voisinage de la substitution, de maintenir le conjugué ou l'hydrophobicité de la molécule ou la majeure partie de la chaîne latérale.

Les substitutions modifiant le plus les propriétés du récepteur sont notamment
- celles dans lesquelles un reste d'acide aminé, par exemple séryl ou thréonyl est substitué par un reste hydrophobe, par exemple leucyl, isoleucyl, phénylalanyl, alanyl ou valyl ;
- celles dans lesquelles un cystéinyl est remplacé par un reste quelconque ;
- celles dans lesquelles un reste ayant une chaîne latérale électropositive, comme les restes lysyl, arginyl, ou histidinyl, est remplacé par un reste électronégatif, par exemple glutamyl ou aspartyl ;
- et celles dans lesquelles un reste ayant une chaîne latérale volumineuse, par exemple phénylalanyl est remplacé par un reste qui en est dépourvu.

Les variantes de substitution peuvent concerner également la structure du récepteur complet de l'interféron et concerneront notamment sa région trans-membranaire. En effet, les substitutions à ce niveau, en réduisant l'affinité dudit polypeptide pour les cellules ou les membranes lipidiques apporteront une forme soluble du récepteur des interférons α et β.

Le domaine trans-membranaire pourra par exemple être substitué par une séquence différente d'acides aminés, par exemple une séquence d'ADN homopolynucléotidique ou une séquence quelconque comprenant de 5 à 50 acides aminés identiques, par exemple sérine, lysine, arginine, glutamine et acide aspartique ou d'autres acides aminés hydrophiles permettant la sécrétion des récepteurs solubles dans le milieu de culture des cellules-hôtes recombinantes.

La présente demande a donc aussi pour objet les polypeptides hydrosolubles, caractérisés en ce qu'ils dérivent par substitution des polypeptides répondant à la formule de la figure 1 ou de la figure 2.

Les données ci-dessus montrent que tant des substitutions ou des délétions que des combinaisons de telles modifications pourront être réalisées.

De manière générale, les variantes ainsi obtenues n'auront pas de domaine trans-membranaire fonctionnel et de préférence n'auront pas de partie intracellulaire (cytoplasmique).

D'autres variantes des polypeptides hydrosolubles ci-dessus décrits pourront être produites par modification chimique, afin notamment d'améliorer les caractéristiques du récepteur des interférons α et β.

De tels polypeptides pourront renfermer des polymères hydrophiles tels que le polyéthylèneglycol greffés sur leurs acides aminés comportant des groupes amino libres, comme la lysine ou des groupes sulfhydryle comme la cystéine.

Ces modifications pourront notamment conférer aux polypeptides selon la présente invention une demi-vie plus élevée dans le plasma ou encore une augmentation de leur solubilité ou enfin une réduction du caractère immunogène desdits polypeptides.

Ces modifications peuvent être réalisées par les méthodes bien connues en elles-mêmes telles que par exemple celles décrites dans le brevet US-4 179 337.

La présente demande a également pour objet les polypeptides ci-dessus décrits, caractérisés en ce qu'ils sont hybridés (ou encore couplés).

Le couplage peut impliquer des polypeptides immuno compétents, par exemple des polypeptides capables de provoquer une réponse immune chez l'animal auquel le polypeptide hybride est administré ou capable de se lier à tout anticorps dirigé contre la partie du polypeptide ne correspondant pas au récepteur soluble des interférons.

De manière générale, les épitopes ne correspondant pas audit récepteur comprendront les antigènes reconnus par les anticorps pré-existants, par exemple des fragments polypeptidiques de bactéries, comme la bétagalactosidase.

Des couplages immuns peuvent être réalisés par liaison croisée in vitro ou par culture cellulaire recombinante transformée par un ADN codant pour un polypeptide immunogène.

Dans des conditions préférentielles, l'agent immunogène sera inséré dans ou lié au récepteur soluble ou un fragment dérivé du récepteur soluble par une liaison polypeptidique de manière à obtenir une chaîne polypeptidique linéaire contenant des épitopes correspondant au récepteur soluble et au moins un épitope étranger audit récepteur. Ces épitopes peuvent être introduits en tout autre emplacement de la chaîne polypeptidique compatible du récepteur ou de ses fragments.

De tels hybrides peuvent être particulièrement utiles lorsqu'une formulation comprenant un support pharmacologiquement acceptable est administrée à un animal en vue de la préparation d'anticorps contre le récepteur de l'interféron ; ces anticorps sont eux-mêmes utiles à titre d'agent de diagnostic ou pour la purification du récepteur natif ou du récepteur soluble de l'interféron.

D'autres polypeptides couplés pouvant être immunogènes comprennent des hybrides contenant en plus du polypeptide hydrosoluble que l'on a couplé à la région C-terminale d'un polypeptide selon l'invention, un homopolymère tel qu'une pentahistidine. On pourra alors facilement isoler l'hybride en employant des agents chélatants tels que des ions de zinc fixés sur un support permettant ainsi d'adsorber l'hybride à partir de mélanges impurs et de l'éluer. Le récepteur soluble peut ensuite être récupéré par exemple par clivage enzymatique.

D'autres hybrides peuvent être réalisés pour améliorer la sécrétion du récepteur soluble. Un polypeptide signal hétérologue remplace alors celui du récepteur soluble et si l'hybride résultant est reconnu par la cellule-hôte, il est mis en oeuvre par la cellule-hôte et le récepteur est sécrété.

La sélection des polypeptides signaux peut être réalisée en se basant sur les caractéristiques de la cellule-hôte employée et peut inclure des séquences de bactéries, de levures, de champignons, de plantes, de mammifères ou de virus.

Dans des conditions préférentielles, les polypeptides ci-dessus décrits sont couplés à des polypeptides, notamment possédant une structure apte à ralentir leur dégradation dans l'organisme humain.

L'hybridation notamment des protéines de plasma ayant une demi-vie plasmatique supérieure à celle de la partie soluble du récepteur lui-même (ordinairement supérieure à 20 heures pour ces protéines plasmatiques) avec un polypeptide soluble selon la présente invention permettra l'allongement de la durée d'action du polypeptide soluble.

De telles protéines plasmatiques comprennent par exemple la sérum-albumine, des apolipoprotéines, des transferrines et de préférence des immunoglobulines, notamment de type G, particulièrement G1.

De préférence, de tels hybrides ne seront pas immunogènes chez l'animal ou chez l'homme chez lesquels ils seront utilisés et lesdites protéines plasmatiques ne procureront pas non plus d'effets secondaires néfastes chez les patients de par leur propre activité biologique habituelle.

Dans des conditions préférentielles de mise en oeuvre, le polypeptide hydrosoluble selon l'invention sera couplé à une immunoglobuline, notamment au niveau de sa région constante. Une immunoglobuline préférée sera de type G, particulièrement G1.

Les immunoglobulines et certaines de leurs variantes sont connues ; beaucoup ont été préparées par culture de cellules recombinantes (Köhler et al, PNAS, USA, 77, 2197 (1980) ; Morrison et al, Ann. Rev. Immunol. 2, 239 (1984)).

Les polypeptides selon la présente invention ayant l'activité des parties extra-cellulaires du récepteur natif des interférons α et β peuvent être couplés par leur extrémité C-terminale à l'extrémité N-terminale de la région constante de la chaîne légère ou de la chaîne lourde.

On peut ainsi remplacer la région variable et on conserve au moins la région charnière CH2 et CH3 de la région constante de la chaîne lourde sous une forme fonctionnellement active.

On peut pour cela construire la séquence ADN appropriée et l'exprimer dans des cellules de cultures recombinantes.

Les immunoglobulines et les autres polypeptides ayant notamment une demi-vie dans le plasma supérieure à celle du récepteur soluble des interférons α et β peuvent être couplés audit récepteur et à ses variantes selon le même procédé.

La partie extra-cellulaire du récepteur de l'interféron comprendra au plus 427 à 436 acides aminés à partir de la méthionine initiale (voir figure 1).

Généralement, les séquences contenant la région cellulaire comprenant la région de fixation seront couplées à la séquence des immunoglobulines.

Le site précis de couplage n'est pas critique. Les frontières indiquées ci-dessus sont uniquement à considérer comme indications et d'autres sites du récepteur soluble de l'interféron avoisinants peuvent être choisis dans le but d'optimiser la sécrétion ou les caractéristiques de fixation du récepteur soluble des interférons α et β. Le site optimal pourra être déterminé par des expérimentations classiques.

En général, il a été trouvé que les hybrides sont exprimés dans la cellule, mais avec une certaine variation dans le degré de sécrétion des cellules-hôtes recombinantes. Le tableau suivant montre différentes fusions immunoglobulines-récepteur de l'interféron qui ont été obtenues :
(a) RCl
(b) (RCl)2
(c) (RCh)2
(d) (RCl)2(RCh)2
dans lesquelles "R" représente une portion du domaine extra-cellulaire du récepteur soluble des interférons α et β contenant le site de fixation et Cl et Ch représentent les domaines constants respectivement de la chaîne légère et de la chaîne lourde de l'immunoglobuline humaine. Les structures ci-dessus représentent seulement les structures principales, par exemple ils ne montrent pas la région joint (J) ou d'autres domaines de l'immunoglobuline, pas plus que les ponts disulfure. Lorsque de tels domaines sont nécessaires à l'activité de liaison, ils doivent être présents dans la position qu'ils occupent naturellement dans le récepteur soluble des interférons α et β, immuno-récepteur des interférons α et β ou immunoglobuline.

Ces exemples sont représentatifs pour des hétéro-anticorps bifonctionnels comprenant des sites ligand-récepteur différents où une immunoglobuline VhCh pourra se lier à un antigène prédéterminé. Des structures plus complexes résulteront en employant des chaînes lourdes d'immunoglobulines d'autres classes, par exemple IgM, IgG2, 3, 4, IgA, IgE, IgD, mais de préférence IgG1.

Un hybride préféré résulte de la fusion de l'extrémité N-terminale du récepteur soluble des interférons α et β qui contient le site de fixation pour le ligand des interférons α et β à la portion C-terminale Fc d'un anticorps qui contient les fonctions effecteur de l'immunoglobuline G1. De tels exemples sont illustrés ci-après dans la partie expérimentale.

De tels hybrides contiennent typiquement les 436 premiers acides aminés ou bien les 427 premiers acides aminés du récepteur soluble des interférons α et β liés par leur extrémité C-terminale à la région constante de la chaîne K ou la chaîne G1. L'ADN codant pour le récepteur soluble des interférons α et β décrit en figure 1 est synthétisé en employant une combinaison de la réaction PCR (Polymerase Chain Reaction : voir R.K. Saiki et al, Science 239, 487-491 (1988) et des digestions par des enzymes de restriction appropriées comme illustré dans les exemples. On a employé des oligonucléotides complémentaires à la séquence d'ADNc à l'extrémité 5' et à des sites prédéterminés en aval de l'ADNc du récepteur d'IFN(a/b) en se basant sur la séquence figurant à la figure 2.

Comme matrice pour la réaction PCR, on a employé un plasmide contenant l'ADNc complet (Uzé et al) ou un bactériophage lambda. Une banque d'ADNc commercialement disponible peut également être utilisée ; on peut aussi synthétiser le fragment d'ADNc directement à partir de l'ARN total ou bien de l'ARN polyA+ préparé selon des méthodes bien connues (voir O. Ohara et al, PNAS 86, 5673-77 (1989), J. Delort et al, Nucl. Acid Res. 17, 6439-6448 (1989)). La banque d'ADNc ou l'ARN peuvent être obtenus de cellules humaines comme Daudi, Namalwa ou d'organes comme la rate humaine avec essentiellement le même résultat.

Le récepteur de l'interféron apparait être un polypeptide unique. Sa séquence présente néanmoins une certaine variation allélique.

Le fragment d'ADN est inséré dans l'ADN codant pour la région- constante de la chaîne légère ou lourde d'une immunoglobuline ; cette dernière sera de préférence une immunoglobuline humaine si la fusion est prévue pour des traitements thérapeutiques chez l'homme.

Des ADN qui codent pour des immunoglobulines sont connus, peuvent être obtenus commercialement ou peuvent être synthétisés (voir par exemple Adams et al, Biochemistry 19, 2711-2719 (1980) ; Gough et al, Biochemistry 19, 2702-2710 (1980) ; Dolby et al, PNAS USA 77, 6027-6031 (1980) ; Rice et al, PNAS USA 79, 7862-7865 (1982) ; Falkner et al, Nature 298, 286-288 (1982) et Morrison et al, Ann. Rev. Immunol. 2, 239-256 (1984)).

Les ADN qui codent pour les hybrides seront de préférence transfectés dans des cellules-hôtes en vue de leur expression.

Si l'hôte produit déjà des chaînes lourdes d'immunoglobuline avant la transfection, il suffit alors de transfecter l'hybride récepteur soluble des interférons α et β-chaîne légère, pour produire un hétéro-anticorps bifonctionnel. De même, si la cellule-hôte exprime déjà une chaîne légère, alors l'ADN qui code pour l'hybride récepteur soluble des interférons α et β-chaîne lourde peut être transfecté pour produire un anticorps bifonctionnel. Les immunoglobulines bifonctionnelles qui contiennent une ou plusieurs chaînes contenant le site de fixation des interférons α et β et une ou plusieurs chaînes contenant des régions variables sont dotées de spécificités doubles, à savoir vis-à-vis des interférons α et β et vis-à-vis d'un antigène prédéterminé. Celles-ci sont produites par les procédés mentionnés ci-dessus ou par des procédés in vitro. Dans ce dernier cas, par exemple, des fragments F(ab)2 de l'hybride sont préparés en accord avec des méthodes connues en soi (voir par exemple brevet des Etats-Unis d'Amérique N° 4 444 878).

Une alternative pour produire des anticorps bifonctionnels consiste à fusionner des cellules B ou des hybridomes qui sécrètent des anticorps ayant la spécificité pour un antigène voulu avec des cellules qui produisent des hybrides de récepteur soluble des interférons α et β-immunoglobuline, par exemple des myélomes. Les anticorps bifonctionnels peuvent être récupérés à partir de surnageants de culture de tels hybridomes.

La présente demande a également pour objet les séquences d'ADN, caractérisées en ce qu'elles codent pour les polypeptides hydrosolubles ci-dessus décrits ou leurs hybrides notamment avec des polypeptides.

Des exemples de polypeptides ayant une haute affinité pour les interférons α et β de mammifères sont par exemple les récepteurs solubles des interférons α et β de primates, les récepteurs solubles des interférons α et β humains, murins, canins, félins, bovins, équins et porcins. Ces séquences d'ADN qui codent pour ces polypeptides ont un certain nombre d'applications. Plus particulièrement, ces séquences ou portions de séquences ou leurs copies synthétiques ou semi-synthétiques peuvent être employées pour cribler d'autres banques d'ADNc ou génomiques humains ou animaux pour sélectionner par hybridation d'autres séquences ADN qui sont semblables au récepteur soluble des interférons α et β. De tels ADN, leurs fragments ou leurs copies synthétiques ou semi-synthétiques représentent des variantes des récepteurs solubles des interférons α et β et peuvent être employés comme matériel de départ pour préparer d'autres variantes par mutation. De telles mutations peuvent ne pas changer la séquence des acides aminés codés par les codons mutés ou au contraire changer les acides aminés. Les deux types de mutations peuvent être avantageux pour la production ou l'usage du récepteur soluble des interférons α et β, conformément à la présente invention. Ces mutations peuvent par exemple permettre une production de haut niveau, une purification plus simple ou une activité de liaison plus élevée.

A titre d'exemple de séquence d'ADN, on peut citer la séquence des nucléotides de 1-1343 de la figure 2.

L'ADN codant pour les variantes du récepteur naturel soluble des interférons α et β se présentera de préférence sous une forme qui permet de l'exprimer dans une unité de transcription sous le contrôle d'éléments de contrôle de transcription et de translation de mammifères, de micro-organismes ou de virus compatibles avec la cellule-hôte envisagée. Après transformation, transfection ou infection de cellules appropriées, de tels vecteurs peuvent permettre l'expression du polypeptide recombinant. Les variantes du récepteur naturel soluble des interférons α et β peuvent être exprimées dans des cellules de mammifères, de levures, de bactéries ou d'autres cellules sous le contrôle d'un promoteur approprié. Des vecteurs de clonage et d'expression pour l'emploi avec des hôtes de bactéries, de champignons, de levures ou de cellules de mammifères sont décrits par Pouwels et al, (Cloning Vectors : A laboratory manuel, Elsevier, New York, 1985) dont les parties pertinentes sont mentionnées ici à titre de référence. Le vecteur d'expression peut, mais ne doit pas, porter un site de réplication ainsi qu'une ou plusieurs séquences de marqueurs de sélection qui permettent une sélection dans les cellules transformées.

Les variations introduites dans l'ADN codant pour les polypeptides ci-dessus décrits ne doivent pas changer le cadre de lecture et ne doivent pas créer de séquences complémentaires qui pourraient produire des structures secondaires néfastes à l'expression.

Les polypeptides variants du récepteur naturel soluble de l'interféron auront sensiblement la même activité de liaison que le récepteur naturel, bien qu'il soit possible de sélectionner des variants en vue de changer les caractéristiques du récepteur, par exemple pour les améliorer, comme indiqué ci-dessus.

Alors que le site de mutation est déterminé, la mutation en soi ne l'est pas. Par exemple, pour optimiser la performance d'une mutation à un site donné, le codon ou une région cible pourra être muté au hasard et les polypeptides variants obtenus criblés en vue de trouver la combinaison optimale des activités secondaires. Les techniques pour introduire des mutations de substitution à des sites donnés dans un ADN sont bien connues, par exemple le système de M13. L'ADN codant pour le récepteur humain peut être obtenu par tout procédé connu ; sa séquence figure en figure 2.

En général, des procaryotes sont employés pour cloner les séquences des polypeptides ci-dessus décrits, par exemple E. coli 294 (ATCC N° 31446) est particulièrement intéressant. D'autres souches peuvent être employées, notamment E. coli X 1776 (ATCC N° 31537).

Les polypeptides selon la présente invention peuvent être exprimés dans des cellules de bactéries, de levures, de mammifères ou d'autres types de cellules, sous le contrôle d'un promoteur approprié, par exemple des systèmes de procaryotes comme par exemple E. coli peuvent être employés pour exprimer les protéines recombinantes de la présente invention. E. coli est typiquement transformé en employant un dérivé de pBR322 (ATCC 37017) qui est un plasmide dérivé d'une souche de E. coli (Bolivar et al, Gene 2, 95 (1977). pBR322 contient des gènes pour la résistance à l'ampicilline et à la tétracycline et, ainsi, procure des moyens simples pour identifier les cellules transformées. Le plasmide pBR322 ou d'autres plasmides de microbes doivent aussi contenir, ou être modifiés de manière à contenir, des séquences de contrôle d'expression communément employées dans les constructions ADN recombinantes.

De tels éléments de contrôle incluent par exemple le promoteur lactose (lac) (J. Mol. Appl. Genet., 1, 139-147 (1981)) accessible sous le N° ATCC 37121, le promoteur de β-lactamase (Chang et al, Nature 275, 615 (1978)), le promoteur de tryptophane (Miozzari J. Bact. 133, 1457-1466 (1978)) et des promoteurs hybrides comme le tac (H. de Boer et al, PNAS USA 80, 21-25 (1983)) accessible sous le N° ATCC 37138. D'autres exemples représentatifs mais non limitatifs sont les vecteurs commerciaux comme par exemple pKK223-3 qui contient le promoteur trc ou pPL-lambda qui contient le promoteur du phage lambda et le répresseur thermolabile c1857 (Pharmacia Fine Chemicals, Uppsala, Suède).

D'autres promoteurs fonctionnels conviennent. Les séquences d'ADN sont généralement connues ; ainsi on peut les coupler à un ADN codant pour une variante du récepteur soluble des interférons α et β en utilisant des liants ou des adapteurs appropriés. Les promoteurs pour les systèmes de bactéries contiennent en plus une séquence dite de Shine-Dalgarno (SD) liée d'une manière opérationnelle à l'ADN codant pour l'antigène en aval.

La présente invention a également pour objet des vecteurs d'expression pour produire des quantités utiles de variantes de récepteur soluble des interférons α et β purifiés.

Après la transformation d'une souche hôte adéquate et la culture de ladite souche-hôte jusqu'à une densité de culture appropriée, le promoteur sélectionné est déréprimé par des moyens appropriés (par exemple élévation de température ou induction chimique) et les micro-organismes sont remis en culture. Les micro-organismes sont typiquement récoltés par centrifugation, lysés par des moyens physiques ou chimiques et l'extrait est récupéré pour une purification supplémentaire.

Les micro-organismes sont fermentés, par exemple dans un fermenteur de 10 litres en employant des conditions de croissance et d'aération maximale et d'agitation rigoureuse. Un agent anti-mousse est employé de préférence. Les cultures poussent à 30°C dans le milieu de super-induction comme décrit par Mott et al, PNAS USA 82, 88 (1985), on déréprime à une densité de culture qui correspond à une absorption A600 de 5 à 6 en élevant la température à 42°C et on récolte à partir de 2 à 20 heures, de préférence de 3 à 6 heures après le changement de température. La masse de micro-organismes est d'abord concentrée par filtration ou par d'autres moyens et ensuite centrifugée à 10000 G pendant 10 minutes à 4°C, puis on opère une congélation rapide du culot. Des protéines recombinantes produites en culture de bactéries sont isolées par extraction du culot suivi par une ou plusieurs étapes de concentration, de désalage ou de chromatographie sur échangeur d'ions ou d'exclusion.

Les micro-organismes employés pour l'expression des variantes du récepteur soluble des interférons α et β peuvent être lysés par n'importe quelle méthode qui convient, y compris des cycles de congélation-décongélation, par sonication, rupture mécanique ou l'emploi d'agents chimiques. Le récepteur soluble des interférons α et β a une certaine tendance à former des aggrégats qui peut être réduite en réalisant l'extraction et la purification en présence d'un détergent comme le Tween 80 ou le Triton X100.

Pour les polypeptides hydrosolubles selon la présente invention ayant une séquence d'ADN qui ne commence pas par la méthionine, le signal d'initiation résultera en un acide aminé méthionine supplémentaire en amont et qui représente le résidu N-terminal du produit. Alors que de tels produits ayant une méthionine N-terminale supplémentaire peuvent être employés directement dans les compositions et les méthodes de la présente invention, il est normalement préférable d'enlever cette méthionine avant. Des méthodes classiques dans ce domaine sont connues pour enlever soit in vivo ou ex vitro de telles méthionines N-terminales.

Des systèmes de levures, de préférence en employant des espèces Saccharomyces comme S. cerevisiae, communément accessible, peuvent aussi être employés pour exprimer les polypeptides de la présente invention.

En général, des vecteurs de levure utiles contiennent une origine de réplication et un marqueur de sélection permettant la transformation de la levure ainsi que de E. coli, par exemple le gène de résistance à l'ampicilline de E. coli et le gène trpl de S. cerevisiae qui procure un marqueur de sélection pour un mutant de la levure qui ne peut pas pousser dans le tryptophane (accessible sous ATCC N° 44076) et un promoteur obtenu d'un gène surexprimé dans la levure pour induire la transcription d'un gène en amont. Des séquences de promoteur appropriées pour des hôtes de levures incluent le promoteur de la 3-phosphoglycérate kinase ou d'autres enzymes de la glycolyse, le promoteur de la phosphatase acide, par exemple PH05, le promoteur des facteurs de l'accouplement type alpha. D'autres promoteurs de levures pouvant être induits sont des régions de promoteur telle que l'alcool-déshydrogénase-2 (Russell et al, J. Biol. Chem. 258, 2674, 1982). Un peptide signal, par exemple le peptide signal du facteur α permettant la sécrétion d'une protéine hétérologue de la levure peut être inséré entre le promoteur et le gène structural à exprimer en amont (voir Bitter et al, PNAS USA, 82, 5330, 1984). Des méthodes de transformation de la levure sont connues de l'homme de l'art et une technique représentative est décrite par Hinnen et al, PNAS USA 75, 1929 (1978) qui permet de sélectionner des transformants trp+ dans un milieu de sélection contenant 0,67 % de base azotée de levure, 0,5 % d'acides de Casamino, 2 % de glucose, 10 µg/ml d'uracile. Des souches hôtes transformées contenant des vecteurs ayant le promoteur PHO5 peuvent être cultivées d'abord dans une préculture dans un milieu riche et par la suite déréprimées en réduisant la concentration du phosphate inorganique dans le milieu. La souche est cultivée en employant des techniques conventionnelles.

Le polypeptide recombinant est obtenu par extraction du culot cellulaire qui peut être lysé soit par digestion enzymatique avec des glucosidases suivie d'un traitement au détergent ou par des forces mécaniques comme par exemple la French press, suivi par une ou plusieurs étapes de concentration, de désalage, de chromatographie sur échangeur d'ions ou d'exclusion. Dans le cas où le polypeptide est sécrété dans l'espace périplasmique, il est récupéré après traitement à l'aide d'agents chimiques, par exemple l'EDTA qui endommage la couche externe de la membrane et qui permettent la libération du polypeptide recombinant. Au cas où le polypeptide est sécrété dans le milieu de culture, il peut être directement récupéré.

Des cellules de mammifères peuvent aussi être employées pour exprimer les polypeptides selon la présente invention sous le contrôle de promoteurs appropriés. On pourrait aussi employer des systèmes cell-free pour produire les récepteurs solubles des interférons α et β de mammifère employant des ARN dérivées de l'ADN construit dans la présente invention.

Des promoteurs qui contrôlent l'expression dans des cellules-hôtes de mammifères peuvent être obtenus de différentes sources, par exemple de génomes de virus comme polyome, Virus simien SV40, adenovirus, rétrovirus, cytomégalovirus de l'hépatite B ou de promoteurs de mammifères, par exemple le promoteur contenant les sites sensibles à la DNAse I du gène de la globine-béta humaine ou de promoteurs.de virus d'insectes comme le promoteur du polyhédron du système baculovirus. Pour l'expression dans les cellules animales, on préfère employer la région de contrôle dérivée du promoteur majeur tardé de l'adénovirus 2.

Les régions early (précoce) et late (tardé) de SV40 sont isolées convenablement du virus SV40 sous forme de fragment de restriction qui contient l'origine de réplication du virus (voir Fiers et al, Nature 273, 113, 1978).

La région immédiate précoce du cytomégalovirus humain est isolée sous forme de fragment de restriction Hind IIIE (Greenaway P.J. et al, Gene 18, 3556360, 1982). Le promoteur tardé de l'adénovirus-2 est isolé sous forme de fragment de restriction Hind III contenant les map units de 8 à 17 (S. Hu & J.L. Manley, Proc. Natl. Acad. Sciences (USA) 78, 820 à 824, 1981). Des promoteurs eucaryotiques d'origine cellulaire d'espèces parentes sont aussi utiles.

Dans des vecteurs d'expression d'eucaryotes, la transcription est augmentée en insérant en plus du promoteur des séquences contenant des activateurs. Les activateurs sont des éléments d'ADN qui agissent en cis et qui contiennent quelques 10 à 300 bp et augmentent la capacité d'initiation de transcription d'un promoteur. Beaucoup de ces activateurs sont connus pour des gènes de mammifères (globine, élastase, albumine, insuline, etc.). Cependant, en général, on emploie des activateurs de virus infectant des cellules eucaryotiques. Des exemples incluent l'activateur de SV40 du côté tardé de l'origine de réplication (bp 100-270), l'activateur du promoteur immédiat précoce du cytomégalovirus, l'activateur de polyome du côté tardé de l'origine de réplication et l'activateur de l'adénovirus. Les vecteurs d'expression employés dans les cellules eucaryotiques (levures, champignons, insectes, plantes, animal, humain) peuvent aussi contenir des séquences nécessaires pour l'épissage et pour la terminaison de la transcription des facteurs qui peuvent influencer l'expression du mARN. Des vecteurs d'expression contiendront un gène de sélection.

Des exemples de marqueur de sélection pour les cellules de mammifères sont la déhydrofolate réductase (DHFR), la thymidine kinase ou la néomycine. Lorsque de tels marqueurs de sélection sont transfectés dans une cellule de mammifère hôte, la cellule transformée peut survivre si elle est mise sous pression de sélection. En général, deux types de régimes de sélection sont employés. D'une part, l'emploi d'une lignée mutante dont la croissance est dépendante d'un milieu supplémenté de certains ingrédients comme par exemple les cellules CHO DHFR- ou les cellules murines LTK-. Ces cellules ne sont pas capables de pousser sans l'addition de thymidine ou d'hypoxanthine et ces cellules survivent si un gène fonctionnel de DHFR ou TK est introduit par transfection. Ainsi, les cellules non transformées par le gène DHFR ou TK ne pousseront pas dans du milieu non supplémenté.

Par ailleurs, on emploie la sélection dominante qui ne nécessite pas de cellule mutante : par exemple on transfecte et exprime le gène qui rend la cellule transfectée résistante visà-vis d'une substance toxique (voir Southern & Berg, J. Molec. Appl. Genet. 1, 327 (1982) Mulligan & Berg Science, 209, 1422 (1980) ; Sugden et al, Mol. Cell. Biol. 5, 410-413 (1985).

L'augmentation ou la réplication de certaines régions du chromosome de la cellule est appelée amplification et peut être induite en employant un agent de sélection comme par exemple la méthotrexate (MTX) qui inactive la DHFR. L'amplification ou l'augmentation des copies du gène DHFR résulte en une plus grande production de DHFR en face de quantités plus élevées de MTX. Le degré d'amplification augmente avec la concentration de MTX dans le milieu de culture. L'amplification d'un gène voulu est obtenu par cotransfection du gène voulu et du gène de la DHFR qui sont co-intégrés dans le chromosome. Après co-amplification du gène voulu et du gène DHFR, le gène qui code pour la protéine voulue exprime plus de protéine voulue.

Les cellules hôtes préférées pour l'expression des variantes du récepteur soluble d'interféron de la présente invention sont les cellules de mammifères qui incluent les cellules de rein du singe (COS-7, ATCC CRL 1651 et les cellules de hamster chinois, CHO-DHFR-, Urlaub & Chasin, PNAS (USA), 77, 4216, 1980).

Des méthodes de transformations des cellules de mammifères sont bien connues et une méthode préférée est celle décrite par Graham F. & van der Eb (Virology 52, 456-457, 1973) employant la précipitation de l'ADN avec du phosphate de calcium. Une autre méthode est l'électroportation comme décrit par G. Chu et al, Nucl. Acid. Res. 15, 1311-1326, 1987. D'autres méthodes de transfections comme par exemple l'injection dans le noyau ou la fusion avec des protoplastes peuvent aussi être employées.

La construction des vecteurs d'expression contenant les séquences de contrôle et codantes voulues se fait en employant des méthodes classiques bien connues (voir par exemple Maniatis T. et al, Molecular Cloning, 133-134 Cold Spring Harbor 1982 ; Current Protocols in Molecular Biology, édité par Ausubel et al, 1987, publié par Greene Publishing Associates & Wiley-Interscience). Les plasmides ou les fragments d'ADN isolés sont coupés, taillés et à nouveau épissés dans la forme voulue.

Les séquences correctes des plasmides sont déterminées après transformation avec des 'mélanges de ligation dans E. coli HB101 ou E. coli K12 294 (ATCC 31446). Les transformants résistant à l'ampicilline sont sélectionnés. Les plasmides sont isolés des transformants et analysés par des enzymes de restriction ou par détermination des séquences par des méthodes bien connues (voir Messing et al, Nucl. Acid Res. 9, 309 (1981) ou Maxam et al, Methods in Enzymology 65, 499 (1980).

En général, les cellules hôtes sont transformées par les vecteurs d'expression, après quoi elles sont cultivées dans un milieu approprié qui contient des substances pour induire l'expression des promoteurs, pour sélectionner les transformants ou pour amplifier les gènes. Les conditions de culture telles que la température, le pH, etc. sont celles utilisées pour la culture sélectionnée pour l'expression et sont de la compétence de l'homme de l'art.

Les polypeptides selon la présente invention sont isolés et purifiés à partir de surnageants de cellules hôtes recombinantes. Typiquement, les surnageants sont concentrés par ultrafiltration, purifiés par chromatographie sur échangeur d'ions ou par immuno-affinité pour adsorber les polypeptides attendus et les éluer par la suite. L'antigène a une grande tendance à former des aggrégats. Ainsi, un détergent comme le Tween 80, le Triton X100 ou le CHAPS sera avantageusement incorporé pendant la purification. Le produit final sera stabilisé à l'aide d'une protéine comme l'albumine qui peut ou non contenir un détergent.

Il est cependant entendu que tous les vecteurs ou séquences de contrôle d'expression ainsi que toutes les cellules hôtes ne fonctionnent pas de manière égale pour tous les systèmes d'expression. Cependant, l'homme de l'art fera une sélection parmi ces vecteurs, séquences de contrôle d'expression et cellules hôtes, sans sortir du cadre de la présente invention. Par exemple, des hôtes unicellulaires devront être sélectionnés en considérant leur compatibilité avec le vecteur choisi, la toxicité du produit codé par les séquences ADN de la présente invention, les caractéristiques de sécrétion, leurs caractéristiques de folding correct des protéines, leur besoin de fermentation et la facilité de purification des produits recombinants après expression par les séquences ADN selon la présente invention.

Parmi ces paramètres, l'homme de l'art peut sélectionner différentes combinaisons système vecteur/système de contrôle d'expression/cellule hôte qui expriment les séquences ADN selon l'invention en fermentation ou culture de cellules animales à grande échelle, par exemple des cellules CHO ou COS-7.

Les polypeptides produits après expression des séquences d'ADN selon l'invention pourront être isolés de la fermentation ou culture de cellules animales et purifiés par une combinaison de méthodes conventionnelles. L'homme de l'art pourra sélectionner la méthode d'isolement et de purification la plus appropriée sans s'éloigner du cadre de la présente invention.

La présente demande a donc également pour objet les cellules, caractérisées en ce qu'elles expriment un polypeptide ci-dessus décrit et un procédé de préparation desdits polypeptides, caractérisé en ce que l'on cultive une cellule capable d'exprimer ledit polypeptide dans un milieu nutritif approprié.

Les polypeptides selon la présente invention sont utiles notamment comme immunomodulateurs, tout particulièrement immunosuppressants et servent au traitement des maladies auto-immunes et des rejets des greffes.

C'est pourquoi la présente demande a aussi pour objet les médicaments, caractérisés en ce qu'ils sont constitués par les polypeptides hydrosolubles tels que définis ci-dessus.

La présente demande a aussi pour objet les compositions pharmaceutiques, caractérisées en ce qu'elles renferment à titre de principe actif un des médicaments tels que définis ci-dessus.

Les polypeptides purifiés peuvent être formulés sous une forme conventionnelle pharmacologiquement acceptable. Les constituants de cette invention contiennent une quantité immuno-thérapeutiquement efficace d'un polypeptide selon la présente invention et un support pharmaceutiquement acceptable. Les constituants selon la présente invention peuvent se présenter sous différentes formes : solide, semi-solide et liquide, sous formes de tablettes, de pilules, de poudre, de solutions injectables ou infusables. La forme préférée dépend du mode d'administration et de l'application thérapeutique. En général, la composition pharmaceutique de la présente invention sera adaministrée en employant des méthodes et des compositions similaires à celles employées pour d'autres polypeptides pharmaceutiquement importants. Ainsi, le polypeptide peut être préservé sous forme lyophilisée, reconstituée avec de l'eau stérilisée juste avant l'administration et administré par les voies habituelles, c'est-à-dire parentérale, sub-cutanée, intraveineuse, intramusculaire ou intralésionale. Une dose effective peut être de l'ordre de 1 à 5 mg/kg de poids corporel/jour. Il est entendu qu'une dose plus faible ou plus forte dépassant de deux fois la dose supérieure peut être injectée sans effets toxiques.

Les médicaments selon l'invention peuvent être administrés à des patients chez qui la production anormale d'interférons α et β constitue un élément nocif, par exemple dans des maladies telles que le Lupus érythémateux, la maladie de Behcet, l'anémie aplasique, le diabète mellitus, la sclérose en plaques, l'arthrite rhumatoïde ou de graves maladies immunodéficitaires ou chez les patients atteints du SIDA. De plus, les constituants selon la présente invention, de par leur qualité de pouvoir moduler les activités immunes telles que l'activation des cellules NK ou l'expression des antigènes d'histocompatibilité seront aussi administrés comme traitement thérapeutique à des patients souffrant de rejet des greffes d'organe.

De par leur nature et leur mécanisme d'action, les compositions selon la présente invention sont exemptes de la toxicité générale comme celle des immunosuppresseurs chimiques tels que les glucocorticoïdes et représentent ainsi une grande amélioration par rapport aux médicaments actuellement en application clinique : par exemple des substances ou dérivés de substances immunosuppressantes comme des corticostéroïdes adrénales qui inhibent la division cellulaire et la synthèse de cytokines pour tous les éléments du système immun ou comme la cyclosporine, un undécapeptide cyclique qui inhibe sélectivement l'activation du système immun et représente une nette amélioration mais qui a un grand nombre d'effets toxiques non immuno-logiques (voir N. Engl. J. Med., 321:25, 1725-1738, 1989).

Les constituants selon la présente invention peuvent aussi être employés pour contrôler des agonistes ou des antagonistes qui sont différents des interférons α et β.

Les constituants selon la présente invention sous forme purifiée sont aussi utiles pour la détermination de la structure tertiaire du site de fixation du récepteur soluble des interférons α et β, une condition préalable pour faire de la conception moléculaire pour en déduire des structures servant de modèle pour la synthèse des antagonistes utiles thérapeutiquement comme agent immunosuppressant ou agonistes utiles comme agent antiviral et antitumoral.

La présente demande a donc enfin pour objet l'application d'un polypeptide tel que défini ci-dessus, à titre d'agent de diagnostic ainsi qu'à la préparation d'anticorps anti-interférons α ou β.

Les exemples qui suivent illustrent la présente invention, sans toutefois la limiter.

### Exemple 1

### Construction de vecteurs pour exprimer le récepteur soluble des interférons α et β natif et des variantes sécrétées de celui-ci dans des cellules de mammifères.

### Section 1

### Construction de plasmides pour exprimer l'interféron natif

Des fragments d'ADN contenant l'ADNc complet et qui codent pour le récepteur des interférons α et β natif ont été synthétisés en employant la réaction PCR. Un bactériophage lambda ZAP qui contient la longueur complète de l'ADNc du récepteur des interférons α et β décrit à la figure 2 a servi de matrice et des oligonucléotides spécifiques d'amorceurs pour la réaction. En particulier, le bactériophage lambda ZAP a été incubé avec une paire d'oligonucléotides synthétiques consistant en un oligonucléotide complémentaire au brin antisense à l'extrémité 5' de l'ADNc et qui présente la séquence : oligo O:5'-CCGGCTGCAGGGATCTGCGGCGGCTCCCAG-3' et d'un oligonucléotide complémentaire au brin sense à l'extrémité 3' de l'ADNc et qui présente la séquence :

oligo-1:3'-CAAAAAGTCGTCCTCAATGTGACCATGGCC-5'

Les oligonucléotides sont construits de manière qu'après avoir terminé la réaction PCR, les fragments d'ADN à double brin obtenus contiennent un site de clivage pour l'enzyme de restriction Pstl à l'extrémité 5' et Kpnl à l'extrémité 3'.

La réaction PCR a été réalisée dans un volume de réaction de 100 µl dans du tampon PCR et contenait les amorces à 1 mM chacune, 100 pg de bactériophage lambda ZAP et 1 unité de polymérase Taq. Les conditions de réaction étaient les suivantes : 25 cycles, un cycle comprenant une incubation à 95°C pendant 1 minute (dénaturation), 37-40°C pendant 3 minutes (hybridation) puis 72°C pendant 4 minutes (polymérisation). Après le dernier cycle, la réaction a été poursuivie à 72°C pendant 10 minutes et les échantillons ont été entreposés à 4°C. Les produits de réaction ont été extraits au chloroforme et après précipitation par de l'éthanol, lesdits produits ont été digérés successivement par Kpnl et Pstl. Le fragment de 1,7 kb (fragment 1) a été récupéré après électrophorèse à agarose à bas point de fusion et lié au vecteur d'expression pSVAdpA1.

pSVAdpAl a été construit comme suit :

Les séquences SV40 ont été obtenues à partir du plasmide pSV2DHFR (Subrami et al, Molec. Cell. Biol. 1, 854-864 (1981). Elles comprennent l'origine de réplication, l'activateur et les promoteurs précoce et tardé (fragment Pvull-HindIII) et l'intron de l'antigène t entouré par ses sites d'épissage (donneur et accepteur), ainsi que le site de polyadénylation de la région précoce (fragment Bg1 II-Eco RI).

La région du promoteur major tardé de l'adéno-2 a été obtenu à partir du plasmide pAdD26SVpA (fragment EcoRI-PstI) (Kaufman & Shimke, P.A., J. Mol. Biol. 159, 601-621 (1982). Elle contient le promoteur, le leader tripartite (Zain et al, Cell. 16, 851, 1979) et un site d'épissage hybride qui consiste en un site d'épissage donneur de l'adéno-2 et un site d'épissage accepteur de la région variable d'une immunoglobuline.

Un site de clonage multiple est identique à celui trouvé dans le plasmide pUC18. Il contient des sites de clivage pour PstI, Sal I, Acc I, Hinc II, Xba I, Bam Hi, Xma I, Sma I, Kpnl, Sacl, Ban II, EcoRI.

Le plasmide contient l'origine de réplication de E. coli et le gène de résistance à l'ampicilline et a été dérivé du plasmide pML (Lusky & Botchan, Nature 293, 79-81, 1981).

pSVAd1 a été digéré successivement avec Kpnl et Pst I, ensuite traité à la phosphatase et le fragment (fragment 2) qui contient la majeure partie du plasmide a été isolé par électrophorèse sur agarose à basse température de fusion. Le fragment 2 a été lié au fragment 1 et le mélange de fixation a été transfecté dans des cellules E. coli HB101 compétentes. La culture transformée a été versée sur des boîtes de Pétri contenant dans leur milieu de culture de l'ampicilline et les colonies résistantes à l'ampicilline ont été sélectionnées. De ces transformants, l'ADN plasmide a été préparé et analysé par des enzymes de restriction pour la présence de l'insert correct et par séquençage pour l'analyse des jonctions de l'insert aux extrémités 5' et 3' pour confirmer que la séquence était correcte.

Le plasmide pSVAdIFRpA a été employé pour exprimer le récepteur cellulaire natif dans des cellules de mammifères.

### Section 2

### Construction de plasmides pour exprimer des variantes de récepteurs d'interférons α et β sécrétées

Des fragments d'ADN qui contiennent différentes délétions du récepteur ayant des terminaisons carboxyle différentes et qui codent pour des formes sécrétées du récepteur ont été synthétisés en employant la réaction PCR. Plus spécifiquement, le bactériophage lambda ZAP qui contient l'ADNc complet du récepteur a été incubé avec différentes paires d'oligonucléotides synthétiques servant de fixateurs et qui contiennent un oligonucléotide complémentaire au brin antisense à l'extrémité 5' et présentant la séquence : bout 5'oligo : 5'-CCGGCTGCAGGGATCTGCGGCGGCTCCCAG-3' et un des oligonucléotides suivants complémentaires au brin sense à des positions différentes de l'ADNc et qui présentent les séquences suivantes :

bout 3' oligo 1: 3'-GGTCCTTTATGGAGATTTACTCCATGGCC-5'
bout 3' oligo 2: 3'-TCACTGCGACATACACTCACTCCATGGCC-5'
bout 3'oligo 3: 3'-GTCAGACCTTTGTGCGGAACTCCATGGCC-5'
bout 3' oligo 4: 3'-GTCACACAGAAAGGAGTTTACTCCATGGCC-5'
bout 3'oligo 5: 3'-CTCTGA TGA ATAACAGATACTCCATGGCC-5'

Les oligonucléotides sont construits de telle sorte qu'après avoir complété la réaction PCR, les fragments d'ADN à double brin obtenus contiennent un site de clivage pour l'enzyme de restriction PstI à l'extrémité 5' et Kpnl à l'extrémité 3'.

La réaction PCR a été effectuée dans un volume de réaction de 100 µl dans du tampon PCR et contenait les amorces à 1 µM chacune, 100 pg de bactériophage lambda ZAP et 1 unité de polymérase Taq. Les conditions àe réaction étaient les suivantes : 25 cycles, un cycle comprenant une incubation à 95°C pendant 1 minute (dénaturation), 37-40°C pendant 3 minutes (hybridation) puis 72°C pendant 4 minutes (polymérisation). Après le dernier cycle, la réaction a été poursuivie à 72°C pendant 10 minutes et les échantillons ont été entreposés à 4°C. Les produits de réaction ont été extraits au chloroforme et après précipitation par de l'éthanol, lesdits produits ont été digérés successivement par Kpnl et Pstl. Les fragments désirés ont été récupérés après électrophorèse sur agarose à basse température de fusion. Les longueurs des fragments récupérés étaient approximativement les suivantes :

réaction 1 (bout 5' oligo +bout 3' oligo 1): 1.3 kb fragment 3
réaction 2 (bout 5'oligo + bout 3'oligo 2): 1.3 kb fragment 4
réaction 3 (bout 5' oligo + bout 3'oligo 3): 1.1 kb fragment 5
réaction 4 (bout 5' oligo + bout 3' oligo 4): 0.9kb fragment 6
réaction 5 (bout 5'oligo + bout 3' oligo 5): 0.6 kb. fragment 7

Les fragments d'ADN de 3-7 ont été liés chacun séparément au vecteur d'expression pSVAdpA1.

Le plasmide pSVAd1 a été digéré successivement avec Kpnl et Pst I, ensuite traité à la phosphatase et le fragment (fragment 2) qui contient la majeure partie du plasmide a été isolé par électrophorèse sur agarose à basse température de fusion. Ensuite, le fragment 2 a été lié séparément à chacun des fragments 3 à 7 et le mélange de fixation a été transfecté dans des cellules E. coli HB101 compétentes. Les cultures transformées ont été versées sur des boîtes de Pétri contenant un milieu de croissance, de l'ampicilline, et les colonies résistantes ont été sélectionnées. De ces transformants, l'ADN plasmide a été préparé et analysé par des enzymes de restriction pour la présence de l'insert correct et par séquençage pour l'analyse des jonctions de l'insert aux extrémités 5' et 3' pour confirmer que la séquence était correcte.

Les plasmides ont été appelés pSVAdsIFlpA, pSVAdsIFR2pA, pSVAdsIFR3pA, pSVAdsIFR4pA, pSVAdsIFR5pA. Ils ont été employés pour exprimer le récepteur soluble et des fragments du récepteur soluble dans des cellules de mammifères.

### Exemple 2

### Expression dans des cellules CHO

Une fois l'expression transitoire du récepteur soluble testée, on a établi des lignées de cellules stables qui expriment de manière continue le récepteur soluble. Pour ce faire, on a employé comme hôte cellulaire la lignée déficiente en déshydrofolate réductase : la lignée CHO DUK-X (F. Kao et al, PNAS USA, 64, 1284-91, 1969 ; Chasin L., & Urlaub G., Proc. Natl. Acad. Sci. 77, 4216-80, 1980). En utilisant ce système, chacune des constructions de récepteur soluble des interférons α et β a été cotransfectée avec pSV2DHFR qui contient le gène DHFR de la souris (Subrami et al, Molec. Cell. Biol. 1, 854-864, 1981). Avant de réaliser cette cotransfection, tous les plasmides ont été linéarisés par clivage avec une enzyme de restriction et préalablement à la transfection, chaque plasmide a été mélangé séparément avec le plasmide pSV2DHFR pour que le rapport molaire de SV2DHFR au plasmide IFR soit de 1:10. Ceci maximalise le nombre de copies des gènes de IFR par transfectant. Les plasmides sont liés dans la cellule pour former des polymères qui peuvent être intégrés dans le chromosome de l'hôte cellulaire par recombinaison (Haynes & Weissmann, Nucl. Acids. Res. 11, 687-706, 1983 ; Scahill S.J. et al, Proc. Natl. Acad. Sci. USA, 80, 4654-58, 1983). On a sélectionné des transfectants qui expriment la DHFR murine dans un milieu α-, milieu de culture sans nucléosides. On a ensuite ajouté du méthotrexate (MTX) (analogue toxique de folate qui se lie à la DHFR) afin de sélectionner des cellules qui expriment des niveaux élevés de DHFR. La résistance à la MTX est due à une expression élevée de DHFR et est souvent le résultat d'une amplification du gène de la DHFR qui peut comprendre de longs segments chromosomiques, appelés "unités amplifiées" (Kaufman & Sharp, Molec. Cell. Biol. 1, 1069-1076, 1981). Ainsi la co-intégration des séquences de DHFR et des récepteurs solubles des interférons α et β permet l'amplification des gènes des récepteurs solubles des interférons α et β.

Des lignées de cellules transfectées de manière stable ont été isolées par clonage dans du milieu de croissance sélectif (DMEM/HAM F12 1:1 (Gibco) supplémenté avec 10 % de sérum de veau foetal. Ensuite, on a criblé les clones pour trouver celui qui exprime le plus de récepteurs solubles des interférons α et β par immunoprécipitation du milieu conditionné après marquage avec la ³⁵S-cystéine in vivo. En particulier, on a incubé approximativement 10⁷ cellules CHO cotransfectées soit par pSVAdIFRpA ou PSVAdsIFR1pA ou pSVAdsIFR2pA ou pSVAdsIFR3pA ou pSVAdpA ou pSV2DHFR pendant 5 heures à 37°C avec 100 mCi/ml de ³⁵S-cystéine (Amersham) dans 4 ml de milieu RPMI cys- (Gibco). Après le marquage de telles cellules, 1 ml de milieu conditionné filtré a été ajusté avec 0,5 mM de fluorure de phénylméthylsulfonyle et immunoprécipité comme décrit ci-après. Le précipité a été fractionné par électrophorèse dans des conditions réductrices sur un gel de PAGE de 7,5 % (U.K. Laemmli, Nature, 227, 680-85, 1980).

### Exemple 3

### Purification du récepteur soluble des interférons α et β

Un clone de cellules CHO qui exprime le récepteur soluble des interférons α et β recombinant de la construction pSVAdsIFR3pA a été sélectionné et mis en culture dans 4 bouteilles roller (Becton & Dickinson) dans du milieu DMEM/HAM F12 (1:1), supplémenté avec 1 % de FCS, 1 g/l de glucose et des antibiotiques tels que la streptomycine et la pénicilline (100 mg/ml) et 0,5 mM MTX pendant 8 jours. Le surnageant a été récupéré, filtré à travers une maxi capsule de 0,2 µm (Gelman Sciences) et on a concentré la protéine sécrétée sur une colonne échangeuse d'anions FPLC mono Q (Pharmacia). La colonne est rincée avec un tampon contenant 100 mM Hepes pH7, 10 % de glycérol, 1 mM de fluorure de phénylméthylsulfonyle (PMSF), 400 unités inhibitrices de kallicréine/ml d'aprotinine avant d'être éluée avec un gradient discontinu de NaCl (de 0,2 à 1,0 M). Le récepteur soluble est capable de fixer l'interféron et par conséquent la fixation de l'interféron marqué à l'iode 125 peut être utilisée pour suivre la purification du récepteur.

Le matériel contenant le récepteur est élué de la colonne mono Q dialysé et concentré dans un appareil de type Micro-Prodicon. On utilise une membrane qui retient les protéines de poids moléculaire supérieur à 10000 daltons. Le matériel concentré est déposé sur un gel de 7,5 % de polyacrylamide de 3 mm avec 0,1 % de SDS. En parallèle, un échantillon (environ 10 µg) est déposé à côté, sur le même gel. Après électrophorèse, cette partie du gel est électro-transférée sur une membrane de bifluorure de polyvidinyle (Millipore) et ensuite hybridée avec l'interféron α 8 humain recombinant marqué à l'iode 125. De cette manière, la position du récepteur soluble peut être identifiée après électrophorèse. La partie du gel qui contient le récepteur soluble est ensuite coupée et la protéine récupérée par électro-élution, concentrée par ultrafiltration moléculaire et dialysée afin d'éliminer le SDS.

### Exemple 4

### Construction de plasmides pour exprimer des variantes de récepteurs solubles des interférons α et β fusionnés à la région constante de l'immunoglobuline kappa humaine et à la région constante de l'immunoglobuline gl humaine

### Fusions à la chaîne légère

Des plasmides ont été construits pour exprimer des récepteurs solubles des interférons α et β ayant différentes longueurs de la région extracellulaire fusionnées à la région constante de l'immunoglobuline kappa. Ces plasmides seront appelés ci-après pSVAdsIFR1K, pSVAdsIFR2K et pSVAdsIFR3K.

Le plasmide pSVAdsIFR1K contient la portion N-terminale à partir du codon d'initiation pour la méthionine jusqu'au point de fusion qui après le codon pour la lysine 436, immédiatement suivi par la séquence de la région constante de l'immunoglobuline kappa qui commence au codon pour la thréonine 109 de l'immunoglobuline kappa humaine (Kabat et al ; Hieter, P.A. et al, Cell 22, 197-207, 1980).

De même, le plasmide pSVAdsIFR2K contient la portion N-terminale à partir du codon d'initiation pour la méthionine jusqu'au point de fusion qui est après le codon pour le glutamate 427, immédiatement suivi par la séquence de la région constante de l'immunoglobuline kappa qui commence au codon pour la thréonine 109 de l'immunoglobuline kappa humaine (Kabat et al ; Hieter, P.A. et al., Cell 22, 197-207, 1980).

Ces plasmides ont été construits comme suit :

Le fragment ADN de l'immunoglobuline kappa a été synthétisé à partir d'une banque d'ADNc de la rate humaine (Clontech Laboratories, Inc.). Pour synthétiser l'ADNc voulu, on a employé des oligonucléotides comme amorce pour la réaction PCR qui ont la séquence complémentaire aux régions prédéterminées, en se basant sur la séquence ADN publiée (Hieter, P.A. et al, Cell 22, 197-207, 1980). L'oligonucléotide de l'extrémité 5' avait la séquence : 5'-ACTGTGGCTGCACCATCTGTCTTCA-3' et celui de l'extrémité 3' avait la séquence :

3'-CCCTCTCACAATCTCCCTCCATGGCCAG-5'

La réaction PCR a été conduite comme décrit à l'exemple 1, excepté qu'on a utilisé 1 µg de plasmide comme matrice et qu'après la réaction, le mélange a été traité à la polymérase Klenow en présence des 4 nucléosides triphosphates pour réparer les extrémités des deux brins. L'ADN a été digéré avec l'enzyme de restriction Kpnl et le fragment voulu a été isolé par électrophorèse sur agarose à basse température de fusion.

Les fragments d'ADN codant pour les fragments de récepteurs solubles des interférons α et β ont été synthétisés de manière identique. L'oligonucléotide de l'extrémité 5' était le même que celui décrit dans l'exemple 1, à savoir : 5'-CCGGCTGCAGGGATCTGCGGCGGCTCCCAG-3' et les oligonucléotides de l'extrémité 3' étaient les suivants :

3'-CTCTTTTGTTTTGGTCCTTTATGGAGATTT-5' oligo 1
3'-TCGTCACAAAAATCACAGCGACATACACTC-5' oligo 2
3'- CCACGAGGTTTTGTCAGACCTTTGTGCGGA-5' oligo 3

Les réactions de PCR ont été faites comme décrit dans l'exemple 1, excepté qu'après le dernier cycle de réaction, les produits de réaction ont été traités à la polymérase Klenow pour réparer les extrémités des deux brins et ensuite avec l'enzyme de restriction Pstl pour obtenir les fragments IFR1, IFR2 et IFR3.

Le vecteur d'expression pSVAdA1 a été digéré successivement par Kpnl et Pstl suivi de phosphatase et le fragment P contenant la plus grande partie du plasmide a été isolé par électrophorèse sur agarose à bas point de fusion.

Dans une réaction comprenant trois composantes, le fragment P a été lié au fragment de l'immunoglobuline et au fragment de sIFR1. De même, le fragment P a été lié au fragment de l'immunoglobuline et au fragment de sIFR2 et enfin le fragment P a été lié au fragment de l'immunoglobuline et au fragment de sIFR3.

Les mélanges de ligation ont été transformés chacun séparément dans des bactéries de E. coli HB101 compétentes et les cultures transformées ont été versées sur des boîtes de Pétri contenant de l'ampicilline dans leur milieu de culture et les colonies résistant à l'ampicilline ont été sélectionnées. Les plasmides ont été isolés des transformants et analysés par des enzymes de restriction pour déterminer l'insert ou par séquençage pour confirmer la séquence ADN de la jonction de l'insert. Ces plasmides ont été utilisés pour exprimer les fusions du récepteur soluble des interférons α et β.

### Fusions de la chaîne lourde

Des plasmides ont été construits pour exprimer des récepteurs solubles des interférons α et β ayant différentes longueurs de la région extracellulaire fusionnées à la région constante de l'immunoglobuline gl. Ces plasmides seront appelés ci-après pSVAdsIFRlgl, pSVAdsIFR2gl et pSVAdsIFR3g1.

Le plasmide pSVAdsIFRlg1 contient la portion N-terminale à partir du codon d'initiation pour la méthionine jusqu'au point de fusion qui se trouve après le codon pour la lysine 436, immédiatement suivi par la séquence de la région constante de l'immunoglobuline gl qui commence au codon pour l'alanine 113 de l'immunoglobuline gl humaine (Kabat et al ; Ellison J.W. et al, Nucl. Acids Res. 10, 4071-4079, 1982).

De même, le plasmide pSVAdsIFR2gl contient la portion N-terminale à partir du codon d'initiation pour la méthionine jusqu'au point de fusion qui est après le codon pour le glutamate 427, immédiatement suivi par la séquence de la région constante de l'immunoglobuline gl qui commence au codon pour l'alanine 113 de l'immunoglobuline gl humaine (Kabat et al ; Ellison J.W. et al., Nucl. Acid Res. 10, 4071-4079, 1982).

Le plasmide pSVAdsIFR3g1 contient la portion N-terminale à partir du codon d'initiation pour la méthionine jusqu'au point de fusion qui se trouve après le codon pour la proline 360, immédiatement suivi par la séquence de la région constante de l'immunoglobuline gl qui commence au codon pour l'alanine 113 de l'immunoglobuline gl humaine (Kabat et al ; Ellison J.W. et al., Nucl. Acid Res. 10, 4071-4079, 1982).

Ces plasmides ont été construits comme suit :

La séquence codant pour l'immunoglobuline G1 a été synthétisée à partir d'une banque d'ADNc de la rate humaine (Clontech Laboratories, Inc.). Pour synthétiser l'ADNc voulu, on a employé des oligonucléotides comme fixateur pour la réaction PCR qui ont la séquence complémentaire aux régions prédéterminées, en se basant sur la séquence ADN publiée (Ellison et al, Nucl. Acid Res. 10, 4071-4079, 1982). L'oligonucléotide de l'extrémité 5' avait la séquence : 5'-GCCTCCACCAAGGGCCCATCGGTCTTCCCC-3' et celui de l'extrémité 3' avait la séquence :

3'-GACAGAGGCCCATTTACTCACCATGGCCAG-5'

La réaction de PCR a été exécutée comme décrit ci-après :

Les fragments sIFR1, sIFR2 et sIFR3 étaient les mêmes que ceux décrits ci-dessus et les vecteurs d'expression ont été construits comme décrit ci-dessus en ce qui concerne les fusion de la chaîne légère.

### Exemple 5

### Construction de vecteurs pour exprimer des variantes du récepteur soluble d'interféron α et β dans E. coli.

On a employé le vecteur d'expression pHR148 pour l'expression du sIFR dans E. coli. pHR148 a été décrit dans Rink et al, Nucl. Acid. Res. 12, 6369-6387, 1984. Le plasmide HR148 a été digéré successivement par les enzymes de restriction Ncol suivi par la polymérase Klenow en présence des 4 nucléosides triphosphates pour réparer les extrémités des deux brins et par Kpnl. Après traitement à la phosphatase, le fragment d'ADN (fragment 1) qui comprend la plus grande partie du plasmide a été isolé par électrophorèse sur agarose à bas point de fusion. Le fragment d'ADN codant pour le récepteur des interférons α et β soluble mature, c'est-à-dire n'ayant pas le peptide signal, a été synthétisé par la réaction PCR en employant le plasmide contenant l'ADNc complet (Uzé et al, cell.) comme matrice et une paire d'oligonucléotides comme amorce. L'oligonucléotide de l'extrémité 5 (dérivé de la région N-terminale de l'interféron) avait la séquence : 5'-GGTGGAAAAAATCTAAAATCTCCTCAAAAG-3' (oligo 1) et l'oligonucléotide de l'extrémité 3' (dérivé de la région qui précède juste la transmembrane de l'interféron avait la séquence :

3'-TCACTGCGACATACACTCATCCCATGGCC-5' (oligo 2).

Les oligonucléotides ont été choisis de manière qu'après la fusion PCR, le fragment synthétisé contienne un site d'enzyme de restriction Kpnl à l'extrémité 3' et un bout "blunt" à l'extrémité 5'.

La réaction PCR a été réalisée comme décrit dans l'exemple 1, sauf qu'après la réaction, les produits ont été traités à la polymérase Klenow pour réparer les extrémités des deux brins et ensuite avec l'enzyme KpnI. Le fragment de 1,3 kb a été isolé par électrophorèse dans de l'agarose à bas point de fusion et ligué au fragment 1.

Un deuxième fragment d'ADN a été synthétisé de manière semblable, sauf que l'oligonucléotide de l'extrémité 5' a été remplacé par la séquence :

5'-GGAAAAAATCTAAAATCTCCTCAAAAAG-3'

### Les produits de ligation ont été transformés dans E. coli HB101 et les cultures transformées ont été versées sur des boîtes de Pétri contenant de l'ampicilline dans leur milieu de culture. Les colonies résistantes à l'ampicilline ont été sélectionnées et les plasmides ont été isolés des transformants et analysés par digestion avec des enzymes de restriction et par séquençage pour confirmer la séquence au point de fusion de l'insert.

Ces deux plasmides appelés ptrpIFRl et ptrpIFR2 ont été employés pour exprimer le récepteur soluble dans E. coli. Pour exprimer la protéine recombinante, les transformants ont été placés dans des milieux de culture de 20 à 50 ml dans du milieu M9 (Rink et al, Nucl. Acid Res. 12, 6369-6387, 1984) pour atteindre une absorption (A650) de 1,0. Ensuite, les cellules ont été centrifugées, lavées et le récepteur soluble de l'interféron a été extrait des cellules brisées et purifié. Le récepteur soluble de l'interféron α et β a été dosé soit par Western blots soit par dot blots comme décrit ci-après.

### Exemple 6

### Méthodes de dosage du récepteur soluble de l'interféron humain α

Les méthodes de dosage du récepteur soluble sont basées sur la capacité du récepteur de fixer d'une façon spécifique l'interféron humain α.
**a) Immunoprécipitation**
   Le dosage du récepteur soluble des interférons humains α et β est basé sur la formation d'un complexe entre le récepteur soluble et l'interféron humain α8 marqué à l'iode 125 qui empêcherait la fixation de ¹²⁵I-IFN à un anticorps monoclonal dirigé contre la partie amino terminale de l'interféron α2 (H. Arnheiter et al, Proc. Natl. Acad. Sci. (USA) 80:2539 [1983]). La partie amino terminale de la molécule n'est pas accessible à l'anticorps après la fixation de l'interféron à son récepteur (H. Arnheiter et al, [1983]). L'¹²⁵I-IFN restant libre est complexé avec l'anticorps anti-IFN puis précipité par l'addition de la protéine A-Sépharose (Pharmacia). L'anticorps anti-IFN est couplé à la protéine A selon les indications du fabricant.
   Les dilutions successives de récepteurs solubles à doser dans 50 µl de tampon boraté pH 8,3 (0,1M H₃BO₃, 0,025M Na₂B₄O₇, 0,075 M NaCl, 0,1 % NP40) sont mélangées avec une quantité constante (75 pmoles) d'interféron a2 marqué à l'iode 125 à une activité spécifique de 81 Bq par fmole (K.E. Mogensen et G. Uzé, Methods in Enzymol. 119C:267 [1986]) et incubées à 4°C pendant une heure. Une quantité constante de protéine A-anti-IFN est reprise dans un volume de 50 µl, auquel on ajoute 50 µl de tampon boraté pH 8,3 (0,2M H₃BO₃, 0,05 M Na₂B₄O₇, 0,15 M NaCl, 0,1 % NP40). Le mélange est agité pendant une heure à 4°C et ensuite centrifugé dans une micro-centrifugeuse de type Eppendorf à 10 000 x g pendant 1 minute, et le surnageant est éliminé. Le précipité est ensuite lavé six fois avec un tampon boraté pH 8,3 (0,1 M H₃BO₃, 0,025 M Na₂B₄O₇, 1 M NaCl, 0,5 % NP40), deux fois avec HEPES 40 mM pH 8 et 2 % de glycérol, puis une fois avec HEPES 40 mM pH 8 et de l'urée 1M, et enfin deux fois avec HEPES 40 mM, pH 8. Le comptage de la radioactivité du complexe immun dans le précipité permettra, en référence à la courbe d'inhibition de formation du complexe interféron-anti-interféron, de déterminer la quantité de récepteur soluble des interférons α et β de l'échantillon ainsi testé.
**b) Dosage du récepteur soluble des interférons humains α et β par inhibition de la fixation de l'interféron a marqué sur son récepteur cellulaire**
   Les dilutions successives de récepteurs solubles des interférons α et β sont incubées à 4°C pendant 30 minutes avec des concentrations variables (0, 50, 100, 125, 500, 1000 et 2000 UI/ml) d'interféron α8 humain recombinant marqué à l'iode 125 (radioactivité spécifique 25 Bq par fmole). Ensuite des cellules lymphoïdes humaines Daudi sont ajoutées à une concentration finale de 10⁷ cellules/ml aux différents mélanges de ¹²⁵I-IFN récepteur soluble et incubées à 4°C pendant deux heures. Les cellules sont alors centrifugées (800 g pendant 10 minutes) et le surnageant est éliminé. Elles sont lavées trois fois avec du milieu RPMI 1640 contenant 0,5 % de sérum de veau foetal (Flow Laboratories) et la radioactivité liée aux cellules est déterminée par comptage avec un compteur gamma de type LKB 1270.
   La quantité de récepteur soluble des interférons α et β est déterminée à partir de la courbe d'inhibition de fixation spécifique de l'interféron à son récepteur cellulaire. (La fixation spécifique est la différence entre la courbe de fixation de ¹²⁵I-IFN seul et celle en présence d'un excès de 100 fois d'interféron non marqué).
**c) Dosage du récepteur soluble des interférons humains α et β par "Western blot"**
   Les échantillons à doser pour la présence du récepteur de l'interféron α sont concentrés par ultrafiltration moléculaire en utilisant une membrane de type Amicon Centricon 30 qui retient les protéines de poids moléculaire supérieur à 30 000 daltons. Les échantillons sont ensuite repris dans un tampon 60 mM Tris-HCl pH 6,8, 1,25 % SDS, 1 mM PMSF et 400 unités inhibitrices de kallicréine/ml d'aprotinine et déposés sur un gel de 7,5 % de polyacrylamide avec 0,1 % SDS. Après électrophorèse dans les conditions standard, les échantillons sont ensuite transférés sur une membrane de bifluorure de polyvinidyle (Millipore) dans un appareil d'électrotransfert de type "semi-dry" (Nova Blot, LKB) à 200 mA pendant une heure dans un tampon 39 mM de glycine, 48 mM Tris-base pH 8,3 et 18 % de méthanol. Après le transfert, la membrane est traitée avec un tampon 20 mM Tris-base, 0,2 % de Tween 20, pH 7,8 et 10 % de lait écrémé (Régilait, France) pendant 4 heures à 25°C sous agitation. Elle est alors hybridée avec du ¹²⁵ I- Interféron α8 à 10⁻¹⁰ M (activité spécifique 50 Bq par fentamole) pendant 2 heures à 25°C sous agitation dans le même tampon. La membrane est ensuite lavée cinq fois avec des aliquots du même tampon, elle est alors séchée à l'air et exposée contre un film sensible aux rayons X.
   L'interféron α8 (αB) humain recombinant est marqué à l'iode 125 par une modification de la méthode à la chloramine T décrite précédemment (K.E. Mogensen et G. Uzé, Methods in Enzymol. 119C:267 [1986]). Ceci correspond à une radioactivité spécifique de 25 Bq par fmole ou 6 Bq par unité internationale d'interféron.
**d) Dosage du récepteur soluble des interférons humains α et β par "dot blot"**
   Les dilutions successives de préparations de réepteurs solubles à doser sont faites dans 50 µl de tampon 39 mM de glycine, 48 mM Tris-base pH 8,3 et déposées dans les godets d'un appareil du type "Bio-Blot" (Biorad) chargé avec une membrane de bifluorure de polyvinidyle (Millipore).
   La membrane est ensuite traitée pendant 4 heures à 25°C sous agitation avec un tampon 20 mM Tris-base, 0,2 % de Tween 20 mM, pH 7,8 et 10 % de lait écrémé (Régilait, France). Elle est alors hybridée avec ¹²⁵I-IFN α8 À 10⁻¹⁰ M (activité spécifique 25 Bq par fmole) pendant 2 heures à 25°C sous agitation dans le même tampon. Ensuite, elle est lavée cinq fois sous agitation avec des aliquots du même tampon et est exposée contre un film sensible aux rayons X. L'analyse des autoradiographies permettra, en référence à une courbe étalon, de déterminer la quantité de ¹²⁵I-IFN attachée à la membrane et donc d'en déduire la concentration du récepteur soluble dans l'échantillon testé.
   L'interféron α8 (αB) humain recombiné est marqué à l'iode 125 par une modification de la méthode à la chloramine T (K.E. Mogensen et G. Uzé, Methods in Enzymol. 119C:267 [1986]). Ceci correspond à une radioactivité spécifique de 25 Bq par fmole ou 6 Bq par unité internationale d'interféron.

### Exemple 7

### Composition pharmaceutique

On a préparé une composition injectable renfermant :
- produit de l'exemple 3 100 mg
- excipient pour préparation injectable 2 ml

## Revendications

1. Polypeptide hydrosoluble caractérisé en ce qu'il a une haute affinité (constante de dissociation inférieure à 10⁻⁹ M) pour les interférons α et β.

2. Polypeptide hydrosoluble selon la revendication 1, caractérisé en ce qu'il répond à la formule de la figure 1.

3. Polypeptide hydrosoluble selon la revendication 1, caractérisé en ce qu'il dérive par substitution du polypeptide répondant à la formule de la figure 1 ou de la figure 2.

4. Polypeptide hydrosoluble selon la revendication 1, caractérisé en ce qu'il dérive par délétion du polypeptide répondant à la formule de la figure 1 ou de la figure 2.

5. Polypeptide hydrosoluble selon la revendication 1, caractérisé en ce qu'il dérive du récepteur naturel de l'interféron α par délétion de la région transmembranaire ou modification de cette région de sorte que cette région transmembranaire ne soit plus fonctionnelle.

6. Polypeptide hydrosoluble selon l'une quelconque des revendications 1 à 5, caractérisé en ce qu'il est hybridé à un autre polypeptide.

7. Polypeptide hydrosoluble selon la revendication 6, caractérisé en ce que l'autre polypeptide possède une structure apte à ralentir la dégradation dans l'organisme humain dudit polypeptide hydrosoluble.

8. Polypeptide hydrosoluble selon la revendication 7, caractérisé en ce que l'autre polypeptide est une immunoglobuline.

9. Polypeptide hydrosoluble selon la revendication 8, caractérisé en ce que l'immunoglobuline est de type G, de préférence G1.

10. Séquences d'ADN, caractérisées en ce qu'elles codent pour le polypeptide selon l'une quelconque des revendications 1 à 9.

11. Cellules, caractérisées en ce qu'elles expriment un polypeptide selon l'une quelconque des revendications 1 à 5.

12. Cellules, caractérisées en ce qu'elles expriment un polypeptide selon l'une quelconque des revendications 6 à 9.

13. Procédé de préparation du polypeptide hydrosoluble selon l'une quelconque des revendications 1 à 9, caractérisé en ce que l'on cultive une cellule capable d'exprimer ledit polypeptide dans un milieu nutritif approprié.

14. Médicament, caractérisé en ce qu'il est constitué d'un polypeptide hydrosoluble selon l'une quelconque des revendications 1 à 9.

15. Compositions pharmaceutiques, caractérisées en ce qu'elles renferment à titre de principe actif un polypeptide hydrosoluble selon l'une quelconque des revendications 1 à 9.

16. Application d'un polypeptide hydrosoluble selon l'une quelconque des revendications 1 à 9 à titre d'agent de diagnostic.

17. Application d'un polypeptide hydrosoluble selon l'une quelconque des revendications 1 à 9 à la préparation d'anticorps anti-récepteurs des interférons α ou β.

## Patentansprüche

1. Wasserlösliches Polypeptid, dadurch gekennzeichnet, daß es eine hohe Affinität (Dissoziationskonstante unterhalb von 10⁻⁹ M) für die Interferone α und β besitzt.

2. Wasserlösliches Polypeptid nach Anspruch 1, dadurch gekennzeichnet, daß es der Formel von Figur 1 entspricht.

3. Wasserlösliches Polypeptid nach Anspruch 1, dadurch gekennzeichnet, daß es durch Substitution aus dem der Formel von Figur 1 oder Figur 2 entsprechenden Polypeptid abgeleitet ist.

4. Wasserlösliches Polypeptid nach Anspruch 1, dadurch gekennzeichnet, daß es durch Deletion aus dem der Formel von Figur 1 oder Figur 2 entsprechenden Polypeptid abgeleitet ist.

5. Wasserlösliches Polypeptid nach Anspruch 1, dadurch gekennzeichnet, daß es vom natürlichen Rezeptor für Interferon α durch Deletion der transmembranen Region oder derartige Modifikation dieser Region, daß diese transmembrane Region nicht mehr funktionell ist, abgeleitet ist.

6. Wasserlösliches Polypeptid nach irgendeinem der Ansprüche 1 bis 5, dadurch gekennzeichnet, daß es mit einem anderen Polypeptid hybridisiert ist.

7. Wasserlösliches Polypeptid nach Anspruch 6, dadurch gekennzeichnet, daß das andere Polypeptid eine Struktur besitzt, die in der Lage ist, den Abbau des wasserlöslichen Polypeptids im menschlichen Organismus zu verlangsamen.

8. Wasserlösliches Polypeptid nach Anspruch 7, dadurch gekennzeichnet, daß das andere Polypeptid ein Immunoglobulin ist.

9. Wasserlösliches Polypeptid nach Anspruch 8, dadurch gekennzeichnet, daß das Immunoglobulin vom Typ G, bevorzugt G1 ist.

10. DNA-Sequenzen, dadurch gekennzeichnet, daß sie das Polypeptid nach irgendeinem der Ansprüche 1 bis 9 codieren.

11. Zellen, dadurch gekennzeichnet, daß sie ein Polypeptid nach irgendeinem der Ansprüche 1 bis 5 exprimieren.

12. Zellen, dadurch gekennzeichnet, daß sie ein Polypeptid nach irgendeinem der Ansprüche 6 bis 9 exprimieren.

13. Verfahren zur Herstellung des wasserlöslichen Polypeptids nach irgendeinem der Ansprüche 1 bis 9, dadurch gekennzeichnet, daß man Zellen züchtet, die in der Lage sind, das Polypeptid in einem geeigneten Nährmilieu zu exprimieren.

14. Medikament, dadurch gekennzeichnet, daß es aus einem wasserlöslichen Polypeptid nach irgendeinem der Ansprüche 1 bis 9 aufgebaut ist.

15. Pharmazeutische Zusammensetzungen, dadurch gekennzeichnet, daß sie als aktiven Bestandteil ein wasserlösliches Polypeptid nach irgendeinem der Ansprüche 1 bis 9 enthalten.

16. Verwendung eines wasserlöslichen Polypeptids nach irgendeinem der Ansprüche 1 bis 9 als diagnostisches Mittel.

17. Verwendung eines wasserlöslichen Polypeptids nach irgendeinem der Ansprüche 1 bis 9 zur Herstellung von Antirezeptor-Antikörpern des Interferons α oder β.

## Claims

1. Water-soluble polypeptide characterized in that it has a high affinity (dissociation constant lower than 10⁻⁹ M) for α and β interferons.

2. Water-soluble polypeptide according to claim 1, characterized in that it corresponds to the formula of Figure 1.

3. Water-soluble polypeptide according to claim 1, characterized in that it is derived, by substitution, from the polypeptide corresponding to the formula of Figure 1 or Figure 2.

4. Water-soluble polypeptide according to claim 1, characterized in that it is derived, by deletion, from the polypeptide corresponding to the formula of Figure 1 or Figure 2.

5. Water-soluble polypeptide according to claim 1, characterized in that it is derived from the natural receptor of α interferon by deletion of the transmembrane region or modification of this region such that this transmembrane region is no longer functional.

6. Water-soluble polypeptide according to any one of claims1 to 5, characterized in that it is hybridized with another polypeptide.

7. Water-soluble polypeptide according to claim 6, characterized in that the other polypeptide has a structure adapted for slowing down the degradation in the human organism of said water-soluble polypeptide.

8. Water-soluble polypeptide according to claim 7, characterized in that the other polypeptide is an immunoglobulin.

9. Water-soluble polypeptide according to claim 8, characterized in that the immunoglobulin is of type G, preferably G1.

10. DNA sequences, characterized in that they code for the polypeptide according to any one of claims 1 to 9.

11. Cells, characterized in that they express a polypeptide according to any one of claims 1 to 5.

12. Cells, characterized in that they express a polypeptide according to any one of claims 6 to 9.

13. Preparation process for a water-soluble polypeptide according to any one of claims 1 to 9, characterized in that a cell is cultivated which is capable of expressing the said polypeptide in an appropriate nutrient medium.

14. Medicament, characterized in that it is constituted by a water-soluble polypeptide according to any one of claims 1 to 9.

15. Pharmaceutical compositions, characterized in that they contain as active ingredient a water-soluble polypeptide according to any one of claims 1 to 9.

16. Use of a water-soluble polypeptide according to any one of claims 1 to 9 as a diagnostic agent.

17. Use of a water-soluble polypeptide according to any one of claims 1 to 9 for the preparation of antibodies against anti-interferon α and β receptors.
